(19) Europäisches Patentamt / European Patent Office / Office européen des brevets

(11) **EP 4 692 144 A1**

(12) **EUROPEAN PATENT APPLICATION**
published in accordance with Art. 153(4) EPC


(43) Date of publication:
**11.02.2026 Bulletin 2026/07**

(21) Application number: **23929958.9**

(22) Date of filing: **17.11.2023**

(51) International Patent Classification (IPC):
***C08F 257/00*** *(2006.01)* ***C08F 257/02*** *(2006.01)*
***C08F 212/36*** *(2006.01)* ***C08F 2/18*** *(2006.01)*
***C08F 2/20*** *(2006.01)* ***C08F 8/36*** *(2006.01)*
***C07D 493/04*** *(2006.01)* ***B01J 31/08*** *(2006.01)*
***B01J 31/10*** *(2006.01)*

(52) Cooperative Patent Classification (CPC):
**B01J 31/08; B01J 31/10; B01J 35/61; B01J 35/63; B01J 35/64; C07D 493/04; C08F 2/18; C08F 2/20; C08F 8/36; C08F 212/14; C08F 212/36; C08F 228/02; C08F 257/00; C08F 257/02**

(86) International application number:
**PCT/CN2023/132168**

(87) International publication number:
**WO 2024/198389 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.03.2023 CN 202310317383**



(71) Applicants:
- **China Petroleum & Chemical Corporation**
  **Beijing 100728 (CN)**
- **Sinopec (Beijing) Research Institute of Chemical Industry Co., Ltd.**
  **Beijing 100013 (CN)**

(72) Inventors:
- **HE, Liming**
  **Beijing 100013 (CN)**
- **DU, Chao**
  **Beijing 100013 (CN)**
- **YI, Zhuo**
  **Beijing 100013 (CN)**
- **WANG, Ruipu**
  **Beijing 100013 (CN)**
- **FENG, Zaixing**
  **Beijing 100013 (CN)**
- **LIU, Qing**
  **Beijing 100013 (CN)**
- **SUN, Zhufang**
  **Beijing 100013 (CN)**
- **XIE, Lunjia**
  **Beijing 100013 (CN)**
- **ZHAO, Siyuan**
  **Beijing 100013 (CN)**

(74) Representative: **Hoffmann Eitle**
**Patent- und Rechtsanwälte PartmbB**
**Arabellastraße 30**
**81925 München (DE)**


(54) **ACIDIC RESIN, PREPARATION METHOD THEREFOR, USE THEREOF, AND PREPARATION METHOD FOR ISOSORBIDE**


(57) The present invention discloses an acidic resin, its preparation method and use, and a catalytic preparation method for isosorbide. The copolymer in the acidic resin comprises a first structural unit derived from styrene having an electron-withdrawing group at the meta position of the benzene ring, a second structural unit derived from divinylbenzene, and a third structural unit derived from an allylsulfonic acid compound represented by

Formula I;

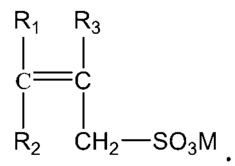


EP 4 692 144 A1

## Description

### Technical Field

The present invention relates to an acidic resin, a preparation method and use thereof, and a catalytic preparation method of isosorbide.

### Background Art

On the one hand, due to the continuous depletion of fossil resources and the resulting environmental pollution, and on the other hand, due to the renewable, abundant, highly functionalized, and environmentally friendly nature of biomass, the development and utilization of biomass raw materials and derivatives has become a hot topic in the fields of fine chemicals and new materials. Isosorbide, a key biomass-derived chemical and a completely non-toxic, green diol, is widely used not only in pharmaceuticals, surfactants, and plastic additives, but also in novel polymer materials. For example, isosorbide is an excellent antihypertensive and diuretic; it can be used to synthesize the surfactants Span and Tween; it can be used to synthesize novel green plasticizers to replace benzoate diester plasticizers; it can be used to modify PET to significantly improve its high-temperature properties and impact resistance; and in new carbonate material technology, it can be used as a raw material to replace the health-prone bisphenol A, thereby improving the environmental performance of polycarbonate. Consequently, the synthesis technology of isosorbide has attracted considerable attention in recent years.

At present, the synthesis technology of isosorbide mainly uses sorbitol or cellulose as raw materials and solid acid or liquid acid as catalyst, wherein the liquid acid is mainly concentrated sulfuric acid, benzenesulfonic acid and ionic liquid etc., and the solid acid mainly comprises molecular sieve, acidic resin, metal oxide, metal phosphate and heteropolyacid etc., and isosorbide is synthesized by batch process. Although batch process has the advantage that investment is low and is suitable for small batch production, there is the shortcomings that product quality is unstable, operation is difficult for realizing automation, so it cannot meet the requirement of large-scale industrial production. Considering the advantage of isosorbide performance and the application in new material field, the market demand of isosorbide may increase rapidly, therefore there is a need to develop the continuous process for preparing isosorbide.

There are already reports of continuous process for producing isosorbide. US6864378B2 uses a continuous process for producing isosorbide, wherein the catalyst is a liquid acid catalyst and the resulting product forms an azeotrope with water vapor and is distilled off. CN104788465A uses a continuous process for producing isosorbide, which uses a two-stage dehydration process, but does not mention the type of the catalyst used, and the product separation requires deacidification and desalination steps. CN204752581U discloses a continuous production apparatus for producing isosorbide from sorbitol. US6639067B1 discloses a continuous process for producing isosorbide, which uses an organic solvent as a water-carrying agent, distills off the product and solvent, and then separates the product, wherein the solvent can be recycled. CN103980286A discloses a continuous process for producing isosorbide, wherein the catalyst is a liquid acid.

The continuous processes described in the aforementioned literature all utilize liquid acid, necessitating neutralization and deacidification during the subsequent separation, as well as subsequent desalination operations, which increases process and production costs. In comparison, solid acid dehydration catalysts offer advantages such as relatively simple product separation and catalyst recyclability. However, solid acid catalysts present challenges in terms of catalyst life and selectivity at high temperatures. In the catalytic dehydration synthesis of isosorbide, solid acid catalysts employ strong acidic resins, which exhibit good conversion and selectivity. However, the catalyst life is limited, particularly at reaction temperatures above 120°C, where the sulfonic acid groups readily detach, significantly decreasing acidity. This fails to meet the requirements of continuous isosorbide production, resulting in high isosorbide production costs and unstable product quality, limiting its industrial application. Furthermore, because the raw material hexitol contains multiple active hydroxyl groups, multiple dehydration reaction pathways occur under the action of the catalyst, wherein the primary dehydration products include 1,4-, 1,5-, and 2,5-dehydration products, while only the 1,4-dehydration product can undergo secondary dehydration reaction to produce isosorbide.

Therefore, it is hoped to provide a new catalyst system that can improve product selectivity and inhibit the formation of by-products, while having long-term stability to meet the requirements of a continuous process.

### Contents of Invention

The lifespan of existing strong acid resin catalysts is limited, making them unable to meet the requirements of continuous isosorbide production processes. This results in high isosorbide production costs and unstable product quality. Furthermore, conventional catalysts in the prior art have poor selectivity for isosorbide, resulting in low isosorbide yields and an inability to simultaneously achieve high catalytic stability, high isosorbide yields, and high selectivity. The present

invention provides an acidic resin, a method for preparing the acidic resin, and the use of the acidic resin as a catalyst for isosorbide production, as well as a continuous method for preparing isosorbide. The acidic resin of the present invention exhibits excellent catalytic performance and stability, particularly high-temperature stability, in the synthesis reaction of isosorbide as a catalyst. In particular, when the acidic resin is used as a catalyst in combination with other solid acid catalysts in the synthesis reaction of isosorbide, not only high catalytic stability is achieved, but also high isosorbide yield and selectivity are achieved. The acidic resin of the present invention is well suited for continuous isosorbide production as a catalyst, thereby reducing production costs and improving product quality stability.

The first aspect of the present invention is to provide an acidic resin, wherein a copolymer in the acidic resin comprises a first structural unit derived from styrene having an electron-withdrawing group at the meta position of the benzene ring, a second structural unit derived from divinylbenzene, and a third structural unit derived from an allylsulfonic acid compound represented by Formula I;

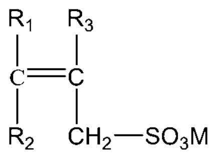 ;

Formula 1

$R_1$, $R_2$ and $R_3$ are each independently hydrogen or C1-C2 alkyl (ie, methyl or ethyl), and M is H or a metal element; and a portion of the benzene ring in the first structural unit carries a $-SO_3H$ group.

In some preferred embodiments of the present invention, the copolymer in the acidic resin contains the first structural unit shown in Formula 2, the second structural unit shown in Formula 3, and the third structural unit shown in Formula 4,

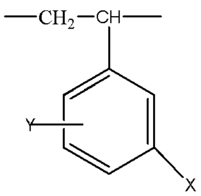

Formular 2 ;

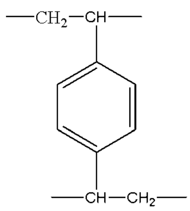

Formular 3 ;

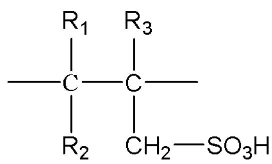

Formular 4 ;

wherein Y in Formula 2 is $-SO_3H$; and X is the electron-withdrawing group; and $R_1$, $R_2$ and $R_3$ in Formula 4 are each independently hydrogen or C1-C2 alkyl.

In the present invention, the acidic resin as a catalyst exhibits excellent catalytic performance and high-temperature stability in the synthesis reaction of isosorbide. In particular, when the acidic resin catalyst and a solid acid catalyst (which is different from the acidic resin catalyst of the present invention) are combined for the synthesis reaction of isosorbide, the acidic resin of the present invention has high catalytic stability and also has high isosorbide yield and high selectivity.

According to the present invention, the electron-withdrawing group can be selected from a wide range. In some preferred embodiments of the present invention, the electron-withdrawing group can be at least one of halogen atom, nitro group, nitroso group, C1-C4 alkoxy, and C1-C4 alkanoyloxy group, such as acetoxy group. The halogen atom can be selected from fluorine, chlorine, bromine, and iodine, for example, fluorine and chlorine.

According to the present invention, when M is a metal element, the metal element can be selected from alkali metals and alkaline earth metals, for example, one or more of lithium, sodium, potassium, magnesium, calcium, strontium and barium. In some embodiments, M can be lithium, sodium or potassium.

According to the present invention, the content of each structural unit in the copolymer can be selected within a wide range. In some embodiments of the present invention, based on the total weight of the copolymer as 100wt%, the content of the first structural unit can be 1-50wt%; the content of the second structural unit can be 30-90wt%; and the content of the third structural unit can be 1-50wt%.

Preferably, in the present invention, based on the total weight of the copolymer as 100wt%, the content of the first structural unit can be 15-40wt%, for example, 15wt%, 20wt%, 25wt%, 30wt%, 35wt%, 40wt%, and a range consisting of any two values; the content of the second structural unit can be 30-70wt%, for example, 30wt%, 35wt%, 40wt%, 45wt%, 50wt%, 55wt%, 60wt%, 65wt%, 70wt%, and a range consisting of any two values; and the content of the third structural unit can be 10-45wt%, for example, 10wt%, 15wt%, 20wt%, 25wt%, 30wt%, 35wt%, 40wt%, 45wt%, and a range consisting of

any two values.

In some embodiments of the present invention, the specific surface area of the acidic resin can be 200-600 $m^2 \cdot g^{-1}$, preferably 200-500 $m^2 \cdot g^{-1}$. For example, the specific surface area of the acidic resin can be 200 $m^2 \cdot g^{-1}$, 250 $m^2 \cdot g^{-1}$, 300 $m^2 \cdot g^{-1}$, 350 $m^2 \cdot g^{-1}$, 400 $m^2 \cdot g^{-1}$, 450 $m^2 \cdot g^{-1}$, 500 $m^2 \cdot g^{-1}$, 550 $m^2 \cdot g^{-1}$, or 600 $m^2 \cdot g^{-1}$, and a range consisting of any two values.

In some embodiments of the present invention, the pore volume of the acidic resin can be 0.2-0.8 $cm^3 \cdot g^{-1}$, preferably 0.2-0.7 $cm^3 \cdot g^{-1}$. For example, the pore volume of the acidic resin can be 0.2 $cm^3 \cdot g^{-1}$, 0.25 $cm^3 \cdot g^{-1}$, 0.3 $cm^3 \cdot g^{-1}$, 0.35 $cm^3 \cdot g^{-1}$, 0.4 $cm^3 \cdot g^{-1}$, 0.45 $cm^3 \cdot g^{-1}$, 0.5 $cm^3 \cdot g^{-1}$, 0.55 $cm^3 \cdot g^{-1}$, 0.6 $cm^3 \cdot g^{-1}$, 0.65 $cm^3 \cdot g^{-1}$, 0.7 $cm^3 \cdot g^{-1}$, 0.75 $cm^3 \cdot g^{-1}$, 0.8 $cm^3 \cdot g^{-1}$, and a range consisting of any two values.

In some embodiments of the present invention, the pore size of the acidic resin can be 1-20 nm, preferably 5-20 nm. For example, the pore size of the acidic resin can be 1 nm, 2 nm, 3 nm, 4 nm, 5 nm, 6 nm, 7 nm, 8 nm, 9 nm, 10 nm, 11 nm, 12 nm, 13 nm, 14 nm, 15 nm, 16 nm, 17 nm, 18 nm, 19 nm, 20 nm, and a range consisting of any two values.

In the present invention, the pore volume, pore size and specific surface area of the acidic resin are measured by nitrogen adsorption-desorption method according to GB/T 19587-2017.

In some embodiments of the present invention, the acid content of the acidic resin can be 1-10 $mmol \cdot g^{-1}$, preferably 3-8 $mmol \cdot g^{-1}$. For example, the acid content of the acidic resin can be 1 $mmol \cdot g^{-1}$, 2 $mmol \cdot g^{-1}$, 3 $mmol \cdot g^{-1}$, 3.5 $mmol \cdot g^{-1}$, 4 $mmol \cdot g^{-1}$, 4.5 $mmol \cdot g^{-1}$, 5 $mmol \cdot g^{-1}$, 5.5 $mmol \cdot g^{-1}$, 6 $mmol \cdot g^{-1}$, 6.5 $mmol \cdot g^{-1}$, 7 $mmol \cdot g^{-1}$, 7.5 $mmol \cdot g^{-1}$, 8 $mmol \cdot g^{-1}$, 9 $mmol \cdot g^{-1}$, 10 $mmol \cdot g^{-1}$, and a range consisting of any two values.

In the present invention, the acid content of the acidic resin is measured by acid-base neutralization titration method with reference to GB/T2895-2008.

The particle size of the acidic resin is not particularly limited in the present invention and any particle size generally known in the art can be used. In some embodiments, the particle size of the acidic resin can be 0.02 to 5 mm, for example, 0.1 to 2 mm.

The acidic resin of the present invention can be used as a catalyst, for example, in reactions that typically use acidic resins as catalysts. In a preferred embodiment, the acidic resin of the present invention is used as a catalyst for the preparation of isosorbide from hexitol. In a particularly preferred embodiment, the acidic resin of the present invention is used as a catalyst for the preparation of isosorbide from the dehydration of 1,4-sorbitan.

The second aspect of the present invention is to provide a method for preparing the acidic resin described in the first aspect, which comprises the following steps:

in the presence of a dispersant and an initiator, divinylbenzene, styrene having an electron-withdrawing group at the meta-position of the benzene ring, and an allylsulfonic acid compound represented by Formula I are copolymerized to obtain a resin matrix;

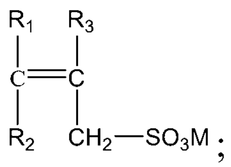

Formula 1

wherein $R_1$, $R_2$ and $R_3$ are each independently hydrogen or C1-C2 alkyl, and M is H or a metal element; and the resin matrix is sulfonated to obtain the acidic resin.

In some preferred embodiments of the present invention, the preparation method comprises the following steps:

(1) a solution containing the dispersant is mixed with a mixed solution containing divinylbenzene, styrene having an electron-withdrawing group at the meta position of the benzene ring, the allylsulfonic acid compound of Formula I, and the initiator, and subjected to polymerization reaction to provide the resin matrix;
(2) the resin matrix is mixed with an organic solvent containing a pore-enlarging agent to obtain a mixed solution containing the resin matrix; and,
(3) the mixed solution containing the resin matrix is contacted with a sulfonating agent for sulfonation, and optionally washed and/or dried to obtain the acidic resin.

According to the present invention, the electron-withdrawing group and M are as described above in the first aspect.

According to the present invention, the amounts of divinylbenzene, styrene having an electron-withdrawing

group at the meta position of the benzene ring, and the allylsulfonic acid compound of Formula I (i.e., the monomers used for copolymerization) can be selected within a wide range. In some embodiments of the present invention, based on the total weight of the copolymer as 100wt%, the content of the first structural unit can be 1-50wt%; the content of the second structural unit can be 30-90wt%; and the content of the third structural unit can be 1-50wt%.

Preferably, in the present invention, based on the total weight of all monomers used to prepare the copolymer as 100wt%, the content of styrene having an electron-withdrawing group at the meta position of the benzene ring can be 15-40wt%, for example, 15wt%, 20wt%, 25wt%, 30wt%, 35wt%, 40wt%, and a range consisting of any two values; the content of divinylbenzene can be 30-70wt%, for example, 30wt%, 35wt%, 40wt%, 45wt%, 50wt%, 55wt%, 60wt%, 65wt%, 70wt%, and a range consisting of any two values; and the content of the allylsulfonic acid compound of Formula I can be 10-45wt%, for example, 10wt%, 15wt%, 20wt%, 25wt%, 30wt%, 35wt%, 40wt%, 45wt%, and a range consisting of any two values.

In the present invention, the copolymerization of divinylbenzene, styrene having an electron-withdrawing group at the meta-position of the phenyl ring, and the allylsulfonic acid compound of Formula I (also referred to as comonomers) can be carried out using methods commonly known in the art for copolymerizing styrene and divinylbenzene, such as free radical suspension polymerization. Free radical suspension copolymerization of styrene and divinylbenzene is known in the art. In a preferred embodiment, the copolymerization of the present invention is free radical suspension polymerization. The copolymerization step of the present invention can employ the same or similar polymerization process and conditions as the free radical suspension copolymerization of styrene and divinylbenzene.

According to the present invention, the dispersant can be selected from a wide range. According to the present invention, a dispersant commonly known in the art for the reaction of step (1) can be used. In some preferred embodiments of the present invention, the dispersant is selected from at least one of polyvinyl alcohol, hydroxyethyl cellulose, and a mixture thereof; preferably, polyvinyl alcohol having a number average molecular weight of 50000 to 100000.

According to the present invention, the initiator can be selected from a wide range. According to the present invention, various initiators commonly used in the art for initiating polymerization of monomers containing vinyl-type functional groups can be used. For example, initiators commonly used in free radical suspension polymerization can be used. Such initiators are known in the art. In some preferred embodiments of the present invention, the initiator can be an azo compound initiator and/or a peroxide initiator, preferably including at least one of benzoyl peroxide, phenylacetyl peroxide, potassium peroxide, and azobisisobutyronitrile.

According to the present invention, the pore-enlarging agent can be selected from a wide range. According to the present invention, the pore-enlarging agent commonly known in the art for step (2) can be used. In some preferred embodiments of the present invention, the pore-enlarging agent can be at least one of dichloromethane, dichloroethane, n-heptane, and mixtures thereof.

According to the present invention, the amounts of the dispersant, the initiator and the pore-enlarging agent can be selected within a wide range.

According to the present invention, those skilled in the art can easily select the amount of the initiator used, as long as the polymerization can be initiated and the resin matrix can be obtained. In some preferred embodiments of the present invention, the amount of the initiator used can be 0.2-6 parts by mass, preferably 0.5-4 parts by mass, relative to 100 parts by mass of all monomers.

According to the present invention, those skilled in the art can easily select the amount of the dispersant used. In some preferred embodiments of the present invention, the amount of the dispersant used can be 1-10 parts by mass relative to 100 parts by mass of all monomers.

According to the present invention, the amount of the pore enlarging agent can be selected within a wide range. In some preferred embodiments of the present invention, the amount of the pore enlarging agent can be 2-40 parts by mass, preferably 2-30 parts by mass, relative to 100 parts by mass of the resin matrix.

According to the present invention, the conditions for the polymerization reaction can be selected within a wide range. According to the present invention, various polymerization conditions commonly used for the polymerization or copolymerization of step (1) can be used, for example, polymerization conditions commonly used in the art for initiating polymerization of monomers containing vinyl-type functional groups, such as free radical suspension polymerization, can be used. In some preferred embodiments of the present invention, the conditions for the polymerization reaction in step (1) can be: a temperature of 60-150 °C, and/or a time of 3-14h. For example, the polymerization temperature can be 60 °C, 70 °C, 80 °C, 90 °C, 100 °C, 110 °C, 120°C, 130 °C, 140 °C, 150 °C or 160 °C, as well as a range consisting of any two values. For example, the polymerization time can be 3 h, 4 h, 5 h, 6 h, 7 h, 8 h, 9 h, 10 h, 11 h, 12 h, 13 h, 14 h, and a range consisting of any two values.

According to the present invention, the conditions for mixing the resin matrix with the organic solvent containing the pore-enlarging agent in step (2) can be selected within a wide range. In some preferred embodiments of the present invention, mixing the resin matrix with the organic solvent containing the pore-enlarging agent in step (2) can include raising the temperature of the mixed system to 30-100 °C at a rate of 0.1-3 °C/min, preferably to 40-100 °C. For example, the heating rate can be 0.1 °C/min, 0.2 °C/min, 0.3 °C/min, 0.4 °C/min, 0.5 °C/min, 0.6 °C/min, 0.7 °C/min, 0.8 °C/min, 0.9

°C/min, 1.0 °C/min, 1.1 °C/min, 1.2 °C/min, 1.3 °C/min, 1.4 °C/min, 1.5 °C/min, 1.6 °C/min, 1.7 °C/min, 1.8 °C/min, 1.9 °C/min, 2.0 °C/min, 2.1 °C/min, 2.2 °C/min, 2.3 °C/min, 2.4 °C/min, 2.5 °C/min, 2.6 °C/min, 2.7 °C/min, 2.8 °C/min, 2.9 °C/min, 3.0 °C/min, and a range consisting of any two values. For example, the temperature can be increased to 30 °C, 40 °C, 50 °C, 55 °C, 60 °C, 65 °C, 70 °C, 75 °C, 80 °C, 90 °C, 100 °C, and a range consisting of any two values.

The sulfonation of the resin matrix is known in the art. The sulfonation step of the present invention can be carried out using conditions generally known in the art for sulfonating polystyrene resins.

According to the present invention, the conditions for sulfonation in step (3) can be selected within a wide range. In some preferred embodiments of the present invention, the conditions for sulfonation in step (3) may include: a temperature of 30-100 °C, and/or a time of 5-20 h. For example, the sulfonation temperature can be 30 °C, 40 °C, 50 °C, 55 °C, 60 °C, 65 °C, 70 °C, 75 °C, 80 °C, 90 °C, 100 °C, and a range consisting of any two values. For example, the sulfonation time can be 5 h, 6 h, 7 h, 8 h, 9 h, 10 h, 11 h, 12 h, 13 h, 14 h, 15 h, 16 h, 17 h, 18 h, 19 h, 20 h, and a range consisting of any two values.

According to the present invention, the sulfonating agent may include a strong acid, such as an acid commonly used in the art for sulfonating polystyrene resins. The type of the strong acid can be selected from a wide range. In some preferred embodiments of the present invention, the strong acid can be selected from at least one of concentrated sulfuric acid, chlorosulfonic acid, oleum, methanesulfonic acid, and mixtures thereof.

After sulfonation, the obtained sulfonated product (sulfonated resin matrix) can be subjected to post-treatment to obtain the acidic resin of the present invention. The post-treatment may include soaking with a solvent, washing, filtering and/or drying. These post-treatment operations are known in the art.

As an example, in an embodiment, the acidic resin of the present invention can be prepared in the following manner:

first, the dispersant and water are added to a reactor, stirred, and slowly heated until dissolved to form a continuous phase; certain proportions of styrene with an electron-withdrawing group at the meta position, divinylbenzene, and the allylsulfonic acid compound represented by Formula I are then mixed and a certain amount of initiator is added. The resulting mixture is then added to the continuous phase. The resulting system is stirred and heated for 5-10 hours to ensure complete polymerization of the monomers. The system is then cooled to room temperature, filtered, washed, dried, and sieved to obtain a mesoporous resin. The prepared mesoporous resin is weighed and added to a reactor, along with the solvent and the pore-enlarging agent. The temperature is slowly raised, and a strong acid is added dropwise to perform sulfonation reaction. After the addition is complete, the reaction is continued for 7-12 hours. After the reaction is complete, the resin is cooled to room temperature, filtered, and separated. The product is then soaked in solvent, washed, filtered, and dried to a constant weight to obtain the acidic resin. The acidic resin of the present invention is prepared by synthesizing the mesoporous granular resin using the substituted styrene, divinylbenzene, and the monomer containing a sulfonic acid group as raw materials, a free radical initiator such as an organic peroxide as the initiator, and a dispersant through suspension copolymerization and sulfonating the mesoporous granular resin.

In some embodiments of the present invention, only divinylbenzene, styrene having an electron-withdrawing group at the meta position of the benzene ring, and the allylsulfonic acid compound of Formula I are used as comonomers to prepare the acidic resin.

The third aspect of the present invention is to provide a use of the acidic resin described in the first aspect or the acidic resin prepared by the preparation method described in the second aspect as a catalyst in the preparation of isosorbide.

In some preferred embodiments, the acidic resin of the present invention may not be used alone as a catalyst for preparing isosorbide; that is, the acidic resin of the present invention can be used in combination with other types of catalysts as the catalyst for preparing isosorbide.

A fourth aspect of the present invention is to provide a method for preparing isosorbide, preferably a continuous preparation method, comprising the steps of:

1) contacting hexitol with a solid acid catalyst to perform a primary dehydration reaction to obtain a primary dehydration reaction product mixture; and
2) removing the solid acid catalyst from the primary dehydration reaction product mixture, and contacting the resulting mixture (i.e., the mixture obtained after removing the solid acid catalyst from the primary dehydration reaction mixture) with the acidic resin of the present invention to perform a secondary dehydration reaction to obtain a reaction product containing isosorbide;

wherein the acidic resin is the acidic resin described in the first aspect and/or the acidic resin prepared by the preparation method described in the second aspect.

The preparation method of isosorbide of the present invention can use hexitol as raw material. The preparation method of the present invention uses a highly selective catalyst, namely the acidic resin of the present invention as a catalyst and is suitable for preparing isosorbide in a continuous process.

The preparation method of the present invention may include a feeding unit, a reaction unit, and a product purification unit, wherein the reaction unit comprises two reactors: a primary reactor prepares a 1,4-dehydrated product, and a secondary reactor prepares isosorbide. Different catalysts can be used in the two-stage reactors depending on the reaction objectives. The primary reactor catalyst is a solid acid catalyst suitable for the production of 1,4-sorbitan, while the secondary reactor uses the acidic resin of the present invention as a catalyst suitable for the production of isosorbide, thereby achieving continuous and efficient production of isosorbide.

The present invention uses the acidic resin of the present invention as a catalyst in combination with a solid acid catalyst and employs a secondary dehydration reaction to produce isosorbide with high yield and high selectivity. Furthermore, the entire preparation method of the present invention is catalytically stable, enabling continuous production.

According to the present invention, hexitol can be selected from a wide range. In some preferred embodiments of the present invention, hexitol is selected from sorbitol and/or mannitol. In the present invention, hexitol can be used in the form of solid hexitol and/or an aqueous solution of hexitol. In some embodiments, the concentration of the aqueous solution of hexitol can be 50-90wt%. For example, the concentration of the aqueous solution of hexitol can be 50wt%, 55wt%, 60wt%, 65wt%, 70wt%, 75wt%, 80wt%, 85wt%, 90wt%, and a range consisting of any two values.

According to the present invention, the solid acid catalyst can be selected from a wide range. In a preferred embodiment, the solid acid catalyst is different from the acidic resin of the present invention. The solid acid catalyst can be a catalyst known in the art for the preparation of isosorbide. In some preferred embodiments of the present invention, the solid acid catalyst can be selected from at least one of a molecular sieve, an acidic resin different from the acidic resin of the present invention, a metal phosphate, a Lewis acid, and a heteropolyacid, preferably at least one of a heteropolyacid and an acidic resin different from the acidic resin of the present invention; more preferably a heteropolyacid, and even more preferably a phosphotungstic acid solid acid catalyst.

The inventors of the present invention have discovered through research that the method for preparing isosorbide of the present invention unexpectedly provides particularly advantageous results, including high yield and selectivity, as well as high catalytic stability, when the phosphotungstic acid solid acid catalyst is used in the primary dehydration reaction and the acidic resin of the present invention is used as a catalyst in the secondary dehydration reaction. It is believed that in the two-stage dehydration reaction of the present invention, the phosphotungstic acid solid acid catalyst and the acidic resin catalyst of the present invention produce a synergistic effect.

According to the present invention, in the primary dehydration reaction, the mass ratio of the solid acid catalyst and hexitol can be selected in a wide range. In some preferred embodiments of the present invention, in the primary dehydration reaction, the mass ratio of the solid acid catalyst and hexitol can be (0.1-1.0): 100, preferably (0.5-6): 100, more preferably (1-5): 100. In the present invention, for the mass ratio of the solid acid catalyst and hexitol, the mass of hexitol before the primary dehydration reaction is used to calculate.

In preferred embodiments of the present invention, the selectivity for 1,4-sorbitan in the primary dehydration reaction is greater than 80%, preferably equal to or greater than 85%, and more preferably equal to or greater than 90%. In these preferred embodiments, isosorbide has a higher yield and selectivity.

According to the present invention, in the secondary dehydration reaction, the mass ratio of the acidic resin catalyst to hexitol can be selected within a wide range. In some preferred embodiments of the present invention, in the secondary dehydration reaction, the mass ratio of the acidic resin catalyst to hexitol can be (1-10): 100, preferably (2-6): 100. In the present invention, for the mass ratio of the acidic resin catalyst to hexitol, the mass of hexitol before the primary dehydration reaction is used to calculate.

In a preferred embodiment of the present invention, the yield of isosorbide prepared by the secondary dehydration reaction is greater than or equal to 79 %, preferably greater than 80 %.

According to the present invention, the conditions in step 1) (primary dehydration reaction) can be selected within a wide range. In some preferred embodiments of the present invention, the conditions in step 1) include: a reaction temperature of 80-130 °C, and/or a time, such as a residence time, of 3-7 h, and/or a reaction pressure of 0.001-1 atm. For example, the reaction temperature can be 80 °C, 90 °C, 100 °C, 105 °C, 110 °C, 115 °C, 120 °C, 125 °C, 130 °C, and a range consisting of any two values. For example, the reaction time can be 3 h, 4 h, 5 h, 6 h, 7 h, and a range consisting of any two values. As will be understood by those skilled in the art, when the method of the present invention is a continuous process, the reaction time can be the residence time. For example, the reaction pressure can be 0.001 atm, 0.005 atm, 0.01 atm, 0.02 atm, 0.03 atm, 0.04 atm, 0.05 atm, 0.06 atm, 0.07 atm, 0.08 atm, 0.09 atm, 0.1 atm, 0.2 atm, 0.3 atm, 0.4 atm, 0.5 atm, 0.6 atm, 0.7 atm, 0.8 atm, 0.9 atm, 1 atm, and a range consisting of any two values.

According to the present invention, the conditions in step 2) (secondary dehydration reaction) can be selected within a wide range. In some preferred embodiments of the present invention, the conditions in step 2) include: a reaction temperature of 120-160 °C, and/or a residence time of 1-9 h, and/or a reaction pressure of 0.001-1 atm. For example, the reaction temperature can be 120 °C, 125 °C, 130 °C, 135 °C, 140 °C, 145 °C, 150 °C, 155 °C, 160 °C, and a range consisting of any two values. For example, the reaction time can be 1 h, 2 h, 3 h, 4 h, 5 h, 6 h, 7 h, 8 h, 9 h, and a range consisting of any two values. As will be understood by those skilled in the art, when the method of the present invention is a continuous process, the reaction time can be the residence time. For example, the reaction pressure can be 0.001 atm,

0.005 atm, 0.01 atm, 0.02 atm, 0.03 atm, 0.04 atm, 0.05 atm, 0.06 atm, 0.07 atm, 0.08 atm, 0.09 atm, 0.1 atm, 0.2 atm, 0.3 atm, 0.4 atm, 0.5 atm, 0.6 atm, 0.7 atm, 0.8 atm, 0.9 atm, 1 atm, and a range consisting of any two values.

In some preferred embodiments of the present invention, the water generated in the primary dehydration reaction is discharged from the top of the reactor during the reaction, and the solid acid catalyst is removed from the primary dehydration reaction product mixture after it is discharged from the reactor, and then it enters the reactor of the secondary dehydration reaction for reaction. For example, the solid acid catalyst can be removed by filtration. In some embodiments, the primary dehydration reaction product mixture can be dehydrated before the secondary dehydration reaction. The dehydrated primary reaction product mixture then enters the reactor of the secondary reaction for reaction.

In some preferred embodiments of the present invention, the preparation method further comprises separating and/or purifying the obtained reaction product containing isosorbide after the secondary dehydration reaction to obtain purified isosorbide.

According to the present invention, the separation and/or purification method can adopt conventional separation and/or purification methods in the art. In some preferred embodiments of the present invention, the separation and/or purification method can be distillation and/or crystallization. Those skilled in the art can easily select the conditions for distillation and/or crystallization. For example, distillation can be carried out at a distillation temperature of about 160 °C and a vacuum degree of about 0.005atm, and then crystallization can be carried out in a mixed solvent of isopropyl alcohol and n-hexane at room temperature, preferably with a mass ratio of isopropyl alcohol to n-hexane of (10-20): 1.

According to the present invention, the method for preparing isosorbide of the present invention can be a batch method or a continuous method, but is preferably a continuous method.

As an example, in one embodiment, the continuous preparation method of isosorbide of the present invention can be implemented in the following manner:

the reaction raw material is hexitol and/or an aqueous solution of hexitol (if hexitol is used, the reaction raw material is added to a preheating tower and heated to have good fluidity), the raw material then enters the first reaction stirred tank, which is equipped with a solid acid catalyst. The reaction temperature is 80-130 °C, the residence time is 3-7h, the reaction pressure is 0.0001-1atm, and the amount of the solid acid catalyst added is 1-10wt% of the reaction raw material (hexitol) by mass. After the reaction is carried out for a certain period of time, the resulting product passes through the reactor valve filter and enters the dehydration tower, while the reaction raw material is replenished at a certain rate. The filtered product is optionally dehydrated through a dehydration tower and then enters the second reaction stirred tank for further dehydration reaction. The second reactor is equipped with the acidic resin of the present invention as a catalyst. The reaction temperature is 120-160 °C, the residence time is 1-9h, the reaction pressure is 0.0001-1atm, and the amount of the catalyst added is 1-10wt% of the reaction raw material by mass. After the reaction is carried out for a certain period of time, the reaction liquid enters a separation system, where the catalyst is removed by filtration. The resulting product mixture is then purified by distillation and crystallization, and dried to obtain the product. Optionally and preferably, a portion of the separated liquid can be recycled. The catalyst can be recycled after filtration and drying, achieving continuous operation of the entire system.

The acidic resin of the present invention exhibits excellent catalytic performance and high-temperature stability when used as a catalyst in the synthesis reaction of isosorbide. In particular, the combination of the acidic resin catalyst and the solid acid catalyst for the synthesis of isosorbide not only achieves high catalytic stability but also exhibits high isosorbide yield and selectivity, making it highly suitable for continuous production, thereby reducing production costs and improving product quality stability.

The present invention uses substituted styrene with an electron-withdrawing group at the meta-position of the benzene ring, divinylbenzene, and the monomer containing a sulfonic acid group represented by Formula I as raw materials. A free radical initiator, such as an organic peroxide, is added as an initiator, and a dispersant is added to synthesize a mesoporous granular resin by suspension copolymerization. The prepared resin is then sulfonated to produce a high-temperature resistant, strongly acidic resin. The sulfonic acid groups in the acidic resin of the present invention have high thermal stability, thereby extending the high-temperature life of the catalyst. Furthermore, by controlling the preparation conditions, the catalyst's pore structure and specific surface area become more favorable for the dehydration reaction, further improving selectivity. Therefore, when the acidic resin catalyst of the present invention is combined with the solid acid catalyst for the synthesis of isosorbide, it exhibits excellent catalytic performance and high-temperature stability, making it suitable for continuous production, thereby reducing production costs and improving product quality stability. The isosorbide prepared by the present invention can be widely used in the fields of new materials, medicine, and organic synthesis.

The present invention utilizes the specific acidic resin catalyst of the present invention and the solid acid catalyst and employs a secondary dehydration reaction to prepare isosorbide with high yield and high selectivity. Furthermore, the preparation method of the present invention is catalytically stable and achieves continuous production.

## Specific Mode for Carrying out the Invention

**Example**

The present invention will be described in detail below with reference to specific examples. However, it should be noted that the following examples are intended only to further illustrate the present invention and are not to be construed as limiting the scope of protection of the present invention. Any non-essential improvements and adjustments made by those skilled in the art based on the present disclosure are still within the scope of protection of the present invention.
Phosphotungstic acid (solid acid catalyst) was analytically pure and purchased from Aladdin Reagent Co., Ltd.
Polyvinyl alcohol was polyvinyl alcohol 1788, analytically pure and purchased from Aladdin Reagent Co., Ltd.
The acid content of the acidic resin was measured by acid-base neutralization titration method with reference to GB/T2895-2008.
The pore volume, pore size, and specific surface area of the acidic resin were measured using the nitrogen adsorption-desorption method according to GB/T 19587-2017.
The pore volume, pore size, and specific surface area were measured using specific surface area and porosity analyzer ASAP 2420 from Micromeritics.
Liquid chromatography was performed using Agilent 1100 high performance liquid chromatograph.
Unless otherwise specified, all raw materials used in the present invention are conventional commercially available products. The raw materials used in the following examples were treated, if necessary, prior to use by methods generally known in the art to meet the requirements of the reaction. For example, styrene and divinylbenzene were treated by distillation to remove polymerization inhibitors prior to use.

**Acidic resin preparation example 1**

2g of polyvinyl alcohol was weighed and added to a three-necked flask, followed by 500mL of water. The mixture was heated to boiling and stirred to dissolve the polyvinyl alcohol completely to form a continuous phase. The mixture was then cooled to 65°C.
30 g of meta-substituted chlorostyrene, 60 g of divinylbenzene and 20 g of sodium allyl sulfonate were weighed in proportion, and 3 g of benzoyl peroxide was weighed and dissolved evenly in a beaker to prepare a dispersed phase.
The dispersed phase was added to the three-necked flask containing the continuous phase, and the mixture was heated to 90°C with stirring for 7 hours to ensure full reaction of the monomers, thereby completing the polymerization reaction. After cooling the polymerization system to room temperature (25°C), it was filtered and separated, washed with deionized water to a pH of 5-7, dried at 100-120°C, and particles within a 10-100 mesh range were collected using a stainless steel sieving machine and weighed to obtain a mesoporous resin.
40g of mesoporous resin was weighed and added to a reactor. 45g of toluene solvent and 8g of ethylene dichloride, a pore-enlarging agent, were added. The temperature was raised to 65°C at a rate of 1.6°C/min. 50g of concentrated sulfuric acid (98% by mass) was then added dropwise. The reaction was continued for 10 hours after the dropwise adding. After the reaction was complete, the system was cooled to room temperature and filtered. The product was then soaked in isopropyl alcohol for 10 hours, washed with water, and filtered. The product was washed with deionized water until neutral, filtered, and dried at 100-120°C to constant weight to obtain the acidic resin.

**Acidic resin preparation example 2**

2g of polyvinyl alcohol was weighed and added to a three-necked flask, followed by 500mL of water. The mixture was heated to boiling and stirred to dissolve the polyvinyl alcohol completely to form a continuous phase. The mixture was then cooled to 65°C.
30 g of meta-substituted chlorostyrene, 60 g of divinylbenzene and 20 g of sodium methyl allyl sulfonate were weighed in proportion, and 3 g of benzoyl peroxide was weighed and dissolved evenly in a beaker to prepare a dispersed phase.
The dispersed phase was added to the three-necked flask containing the continuous phase, and the mixture was heated to 90°C with stirring for 7 hours to ensure full reaction of the monomers, thereby completing the polymerization reaction. After cooling the polymerization system to room temperature, it was filtered and separated, washed with deionized water to a pH of 5-7, dried at 100-120°C, and particles within a 10-100 mesh range were collected using a stainless steel sieving machine and weighed to obtain a mesoporous resin.
40g of mesoporous resin was weighed and added to a reactor. 50g of toluene solvent and 10g of ethylene dichloride, a pore-enlarging agent, were added. The temperature was raised to 65°C at a rate of 1.5°C/min. 50g of concentrated sulfuric acid (98% by mass) was then added dropwise. The reaction was continued for 10 hours after the dropwise adding. After the reaction was complete, the system was cooled to room temperature and filtered. The product was then soaked in isopropyl alcohol for 10 hours, washed with water, and filtered. The product was washed with deionized water until neutral, filtered, and dried at 100-120°C to constant weight to obtain the acidic resin.

### Acidic resin preparation example 3

3g of polyvinyl alcohol was weighed and added to a three-necked flask, followed by 500mL of water. The mixture was heated to boiling and stirred to dissolve the polyvinyl alcohol completely to form a continuous phase. The mixture was then cooled to 65°C.

30 g of meta-substituted chlorostyrene, 60 g of divinylbenzene and 40 g of sodium allyl sulfonate were weighed in proportion, and 3 g of potassium persulfate was weighed and dissolved evenly in a beaker to prepare a dispersed phase.

The dispersed phase was added to the three-necked flask containing the continuous phase, and the mixture was heated to 90°C with stirring for 7 hours to ensure full reaction of the monomers, thereby completing the polymerization reaction. After cooling the polymerization system to room temperature, it was filtered and separated, washed with deionized water to a pH of 5-7, dried at 100-120°C, and particles within a 10-100 mesh range were collected using a stainless steel sieving machine and weighed to obtain a mesoporous resin.

40g of mesoporous resin was weighed and added to a reactor. 60g of toluene solvent and 10g of ethylene dichloride, a pore-enlarging agent, were added. The temperature was raised to 65°C at a rate of 1.6°C/min. 30g of concentrated sulfuric acid (98% by mass) was then added dropwise. The reaction was continued for 10 hours after the dropwise adding. After the reaction was complete, the system was cooled to room temperature and filtered. The product was then soaked in isopropyl alcohol for 10 hours, washed with water, and filtered. The product was washed with deionized water until neutral, filtered, and dried at 100-120°C to constant weight to obtain the acidic resin.

### Acidic Resin Preparation Example 4

3g of polyvinyl alcohol was weighed and added to a three-necked flask, followed by 500mL of water. The mixture was heated to boiling and stirred to dissolve the polyvinyl alcohol completely to form a continuous phase. The mixture was then cooled to 65°C.

25 g of meta-substituted acetoxystyrene, 50 g of divinylbenzene and 40 g of sodium methyl allyl sulfonate were weighed in proportion, and 3 g of potassium persulfate was weighed and dissolved evenly in a beaker to prepare a dispersed phase.

The dispersed phase was added to the three-necked flask containing the continuous phase, and the mixture was heated to 90°C with stirring for 7 hours to ensure full reaction of the monomers, thereby completing the polymerization reaction. After cooling the polymerization system to room temperature, it was filtered and separated, washed with deionized water to a pH of 5-7, dried at 100-120°C, and particles within a 10-100 mesh range were collected using a stainless steel sieving machine and weighed to obtain a mesoporous resin.

40g of mesoporous resin was weighed and added to a reactor. 50g of toluene solvent and 10g of ethylene dichloride, a pore-enlarging agent, were added. The temperature was raised to 65°C at a rate of 1.5°C/min. 30g of oleum (98% by mass) was then added dropwise. The reaction was continued for 10 hours after the dropwise adding. After the reaction was complete, the system was cooled to room temperature and filtered. The product was then soaked in isopropyl alcohol for 10 hours, washed with water, and filtered. The product was washed with deionized water until neutral, filtered, and dried at 100-120°C to constant weight to obtain the acidic resin.

### Comparative Acidic Resin Preparation Example 1

3g of polyvinyl alcohol was weighed and added to a three-necked flask, followed by 500mL of water. The mixture was heated to boiling and stirred to dissolve the polyvinyl alcohol completely to form a continuous phase. The mixture was then cooled to 65°C.

37 g of styrene and 63 g of divinylbenzene were weighed in proportion, and 3 g of benzoyl peroxide was weighed and dissolved evenly in a beaker to prepare a dispersed phase.

The dispersed phase was added to the three-necked flask containing the continuous phase, and the mixture was heated to 90°C with stirring for 7 hours to ensure full reaction of the monomers, thereby completing the polymerization reaction. After cooling the polymerization system to room temperature, it was filtered and separated, washed with deionized water to a pH of 5-7, dried at 100-120°C, and particles within a 10-100 mesh range were collected using a stainless steel sieving machine and weighed to obtain a mesoporous resin.

25g of mesoporous resin was weighed and added to a reactor. 22g of xylene solvent and 14g of ethylene dichloride, a pore-enlarging agent, were added. The temperature was raised to 60°C at a rate of 1.5°C/min. 150mL of concentrated sulfuric acid (98% by mass) was then added dropwise. The reaction was continued for 10 hours after the dropwise adding. After the reaction was complete, the system was cooled to room temperature and filtered. The product was then soaked in isopropyl alcohol for 10 hours, washed with water, and filtered. The product was washed with deionized water until neutral, filtered, and dried at 100-120°C to constant weight to obtain the comparative acidic resin 1.

**Comparative Acidic Resin Preparation Example 2**

The comparative acidic resin was prepared according to the method of Acidic Resin Preparation Example 3, except that the meta-substituted chlorostyrene was replaced by an equal molar amount of styrene monomer, to obtain the comparative acidic resin 2.

**Comparative Acidic Resin Preparation Example 3**

The comparative acidic resin was prepared according to the method of Comparative Acidic Resin Preparation Example 1, except that styrene monomer was replaced by an equal molar amount of meta-substituted chlorostyrene, to obtain the comparative acidic resin 3.

**Isosorbide Synthesis Example 1**

100g of a 70% sorbitol aqueous solution was pumped into the primary dehydration reactor. A solid acid catalyst (phosphotungstic acid solid acid) accounting for 5% of the mass of sorbitol was added as a catalyst of the primary dehydration reaction at the same time. The system was heated to 110°C, stirred, and evacuated to a vacuum of 0.1 atm. Water generated during the reaction was discharged from the top of the reactor via a condenser. After 4 hours of reaction, liquid chromatography revealed a selectivity for 1,4-sorbitan of 95%. A valve was opened to pump the 70% sorbitol aqueous solution into the reactor. At the same time, the reaction solution was passed through a filter at the bottom of the reactor to remove the catalyst. The resulting filtrate was then fed into the secondary dehydration reactor.
The secondary dehydration reactor was loaded with an acidic resin (Acidic Resin Preparation Example 1) accounting for 3% of the mass of sorbitol. After 5 hours of reaction at a temperature of 130°C and a pressure of 0.06 atm, the yield of isosorbide produced by the secondary dehydration reaction was 81.5%, as determined by liquid chromatography. The valve was opened to discharge the reaction solution; the catalyst was removed and recovered by filtration. The obtained product mixture was then purified by distillation (distillation temperature of 160°C, vacuum degree of 0.005 atm) and crystallization (crystallization solvent was a mixed solvent of isopropanol and n-hexane, the mass ratio of the two solvents was 12:1), wherein the crystallization temperature was 20°C. The product was then dried to yield the isosorbide product.

**Isosorbide Synthesis Example 2**

100g of a 75% sorbitol aqueous solution was pumped into the primary dehydration reactor. A solid acid catalyst (phosphotungstic acid solid acid) accounting for 3% of the mass of sorbitol was added as a catalyst of the primary dehydration reaction at the same time. The system was heated to 120°C, stirred, and evacuated to a vacuum of 0.15 atm. Water generated during the reaction was discharged from the top of the reactor via a condenser. After 5 hours of reaction, liquid chromatography revealed a selectivity for 1,4-sorbitan of 93%. A valve was opened to pump the 75% sorbitol aqueous solution into the reactor. At the same time, the reaction solution was passed through a filter at the bottom of the reactor to remove the catalyst. The resulting filtrate was then fed into the secondary dehydration reactor.
The secondary dehydration reactor was loaded with an acidic resin (Acidic Resin Preparation Example 2) accounting for 3.5% of the mass of sorbitol. After 5 hours of reaction at a temperature of 135°C and a pressure of 0.1 atm, the yield of isosorbide produced by the secondary dehydration reaction was 82.4%, as determined by liquid chromatography. The valve was opened to discharge the reaction solution; the catalyst was removed and recovered by filtration. The obtained product mixture was then purified by distillation (distillation temperature of 160°C, vacuum degree of 0.005 atm) and crystallization (crystallization solvent was a mixed solvent of isopropanol and n-hexane, the mass ratio of the two solvents was 15:1), wherein the crystallization temperature was 20°C. The product was then dried to yield the isosorbide product.

**Isosorbide Synthesis Example 3**

100g of a 65% sorbitol aqueous solution was pumped into the primary dehydration reactor. A solid acid catalyst (phosphotungstic acid solid acid) accounting for 1% of the mass of sorbitol was added as a catalyst of the primary dehydration reaction at the same time. The system was heated to 125°C, stirred, and evacuated to a vacuum of 0.06 atm. Water generated during the reaction was discharged from the top of the reactor via a condenser. After 5 hours of reaction, liquid chromatography revealed a selectivity for 1,4-sorbitan of 94%. A valve was opened to pump the 65% sorbitol aqueous solution into the reactor. At the same time, the reaction solution was passed through a filter at the bottom of the reactor to remove the catalyst. The resulting filtrate was then fed into the secondary dehydration reactor.
The secondary dehydration reactor was loaded with an acidic resin catalyst (Acidic Resin Preparation Example

3) accounting for 5% of the mass of sorbitol. After 3 hours of reaction at a temperature of 140°C and a pressure of 0.2 atm, the yield of isosorbide produced by the secondary dehydration reaction was 80.9%, as determined by liquid chromatography. The valve was opened to discharge the reaction solution; the catalyst was removed and recovered by filtration. The obtained product mixture was then purified by distillation (distillation temperature of 160°C, vacuum degree of 0.005 atm) and crystallization (crystallization solvent was a mixed solvent of isopropanol and n-hexane, the mass ratio of the two solvents was 17: 1), wherein the crystallization temperature was 20°C. The product was then dried to yield the isosorbide product.

**Isosorbide Synthesis Example 4**

Isosorbide was synthesized according to the method of Isosorbide Synthesis Example 1, except that the acidic resin prepared in Acidic Resin Preparation Example 1 was replaced by the acidic resin prepared in Acidic Resin Preparation Example 4.

**Isosorbide Synthesis Comparative Example 1**

Isosorbide was synthesized according to the method of Isosorbide Synthesis Example 1, except that the acidic resin prepared in Acidic Resin Preparation Example 1 was replaced by the comparative acidic resin prepared in Comparative Acidic Resin Preparation Example 1.

**Isosorbide Synthesis Comparative Example 2**

Isosorbide was synthesized according to the method of Isosorbide Synthesis Example 1, except that the acidic resin prepared in Acidic Resin Preparation Example 1 was replaced by phosphotungstic acid solid acid catalyst in the primary dehydration reactor. The isosorbide yield was 48.5%.

**Isosorbide Synthesis Comparative Example 3**

Isosorbide was synthesized according to the method of Isosorbide Synthesis Example 1, except that the acidic resin catalyst 1 prepared in Acidic Resin Preparation Example 1 was replaced by the comparative acidic resin prepared in Comparative Acidic Resin Preparation Example 2. The isosorbide yield was 79.6 %. After 10 cycles, the isosorbide yield was 68.1%.

**Isosorbide Synthesis Comparative Example 4**

Isosorbide was synthesized according to the method of Isosorbide Synthesis Example 1, except that the acidic resin catalyst 1 prepared in Acidic Resin Preparation Example 1 was replaced by the comparative acidic resin prepared in Comparative Acidic Resin Preparation Example 3. The isosorbide yield was 80.5 %. After 10 cycles, the isosorbide yield was 65.3%.

The specific surface area, pore volume, pore size and acid content of the acidic resins prepared in the above examples are given in the following table.

| Catalyst | Specific surface area ($m^2 \cdot g^{-1}$) | Pore volume ($cm^3 \cdot g^{-1}$) | Pore size (nm) | Acid content ($mmol \cdot g^{-1}$) |
|---|---|---|---|---|
| Acidic resin preparation example 1 | 439.8 | 0.45 | 14.1 | 4.08 |
| Acidic resin preparation example 2 | 450.1 | 0.39 | 15.2 | 4.62 |
| Acidic Resin Preparation Example 3 | 447.2 | 0.52 | 12.8 | 4.32 |
| Acidic Resin Preparation Example 4 | 436.8 | 0.41 | 13.9 | 4.15 |
| Comparative Acidic Resin Preparation Example 1 | 460.1 | 0.23 | 1.5 | 3.43 |
| Comparative Acidic Resin Preparation Example 2 | 435.2 | 0.38 | 12.6 | 4.52 |
| Comparative Acidic Resin Preparation Example 3 | 451.0 | 0.26 | 4.5 | 4.38 |

**Calculation methods for selectivity and yield**

The selectivity and yield were measured by high performance liquid chromatography and calculated using the following method.

The calculation method for selectivity of 1,4-sorbitan in the primary dehydration reaction:

$$\text{Selectivity of 1,4} - \text{sorbitan} = \frac{moles\ of\ 1{,}4 - sorbitan}{moles\ of\ sorbitol\ before\ reaction\ -\ moles\ of\ sorbitol\ after\ reaction} \times 100\%$$

The calculation method for selectivity of isosorbide in the secondary dehydration reaction:

$$\text{Selectivity of isosorbide} = \frac{moles\ of\ isosorbide}{moles\ of\ sorbitol\ before\ reaction\ -\ moles\ of\ sorbitol\ after\ reaction} \times 100\%$$

The purity of the isosorbide product was determined by high performance liquid chromatography.

The formula for calculating the yield of isosorbide is as follows:

$$\text{Yield of isosorbide} = \frac{moles\ of\ isosorbide}{moles\ of\ sorbitol\ before\ reaction} \times 100\%$$

The purity and the yield of the isosorbide products obtained in the isosorbide synthesis examples and the isosorbide synthesis comparative examples are shown in Table 1.

Table 1

| | Purity of isosorbide product, % | Yield of isosorbide, % |
|---|---|---|
| Isosorbide Synthesis Example 1 | 99.6 | 81.5 |
| Isosorbide Synthesis Example 2 | 99.6 | 82.4 |
| Isosorbide Synthesis Example 3 | 99.5 | 80.9 |
| Isosorbide Synthesis Example 4 | 99.5 | 81.3 |
| Isosorbide Synthesis Comparative Example 1 | 99.2 | 72.0 |
| Isosorbide Synthesis Comparative Example 2 | 99.3 | 48.5 |
| Isosorbide Synthesis Comparative Example 3 | 99.5 | 79.6 |
| Isosorbide Synthesis Comparative Example 4 | 99.5 | 80.5 |

**Stability experiment of acidic resin as catalyst**

The acidic resin catalysts recovered from Isosorbide Synthesis Examples 1-4 and Isosorbide Synthesis Comparative Example 1 were used to prepare 10 batches of isosorbide according to the respective Example and Comparative Example. The purity and yield of the final batch of isosorbide are shown in Table 2. The acid content of the acidic resin recovered in the final batch was measured, and the results are shown in Table 3.

Table 2

| | Purity of isosorbide product, % | Yield of isosorbide, % |
|---|---|---|
| Batch 10 of Isosorbide Synthesis Example 1 | 99.6 | 80.5 |
| Batch 10 of Isosorbide Synthesis Example 2 | 99.6 | 79.7 |
| Batch 10 of Isosorbide Synthesis Example 3 | 99.5 | 80.1 |
| Batch 10 of Isosorbide Synthesis Example 4 | 99.5 | 80.3 |
| Batch 10 of Isosorbide Synthesis Comparative Example 1 | 95 | 30.2 |

Table 3 Comparison of Physical Properties of Acidic Resin Catalyst in Acidic Resin Preparation Example 1

| Catalyst | Specific surface area ($m^2 \cdot g^{-1}$) | Pore volume ($cm^3 \cdot g^{-1}$) | Pore size (nm) | Acid content ($mmol \cdot g^{-1}$) |
|---|---|---|---|---|
| Fresh catalyst (acidic resin preparation example 1, before reaction) | 439.8 | 0.45 | 14.1 | 4.08 |
| Catalyst after 10 repeated batches of reaction | 444.2 | 0.41 | 13.8 | 4.10 |

Table 4

| Type | Fresh catalyst | | After 10 batches | | Fresh catalyst | | After 10 batches | |
|---|---|---|---|---|---|---|---|---|
| | Acid content / ($mmol \cdot g^{-1}$) | Pore structure /(nm) | Acid content /($mmol \cdot g^{-1}$) | Pore size /(nm) | Product yield /% | Product purity / % | Product yield /% | Product purity / % |
| Comparative acidic resin preparation example 1 | 3.43 | 1.5 | 2.18 | 0.8 | 72 | 99.2 | 30.2 | 95 |
| Acidic resin preparation example 1 | 4.08 | 14.1 | 4.10 | 13.8 | 81.5 | 99.6 | 80.5 | 99.6 |

It can be seen from the results in Tables 1 to 4 above, the acidic resin catalyst of the present invention exhibits excellent catalytic performance and high-temperature stability in the synthesis reaction of isosorbide. By combining the acidic resin of the present invention as a catalyst with a solid acid catalyst for the synthesis of isosorbide, not only is high catalytic stability achieved, but also high isosorbide yield and selectivity are achieved.

It should be noted that the examples described above are only for illustration of the present invention and do not constitute any limitation of the present invention. The present invention has been described with reference to typical examples, but it should be understood that the words used therein are descriptive and explanatory words, rather than restrictive words. The present invention can be modified as specified within the scope of the claims of the present invention, and the present invention can be revised without departing from the scope and spirit of the present invention. Although the present invention described therein relates to specific methods, materials and examples, it does not mean that the present invention is limited to the specific examples disclosed therein. On the contrary, the present invention can be extended to all other methods and applications having the same function.

All publications, patent applications, patents, and other references mentioned in this specification are incorporated herein by reference. Unless otherwise defined, all technical and scientific terms used in this specification have the meanings conventionally understood by those skilled in the art. In the event of conflict, the definitions in this specification shall prevail.

When this specification uses the prefix "well known to those skilled in the art", "prior art" or similar terms to introduce materials, substances, methods, steps, devices or components, the objects introduced by the prefix include those commonly used in the art when this application is filed, but also include those that are not commonly used at present but will become generally recognized in the art to be suitable for similar purposes.

The endpoints and any values of the ranges disclosed in this application document are not limited to the precise ranges or values, and these ranges or values should be understood to include values close to these ranges or values. For numerical ranges, the endpoint values of each range, the endpoint values of each range and a separate point value, and the separate point values can be combined with each other to obtain one or more new numerical ranges, and these numerical ranges should be considered as specifically disclosed in the description. Hereinafter, in principle, each technical solution can be combined with each other to obtain a new technical solution, which should also be considered as specifically disclosed in this article.

In the context of this specification, except those explicitly stated, any matters or items not mentioned are directly applicable to those known in the art without any changes.

Moreover, any embodiment described herein can be freely combined with one or more other embodiments described herein, and the technical solutions or technical ideas thus formed shall be deemed as part of the original disclosure or original record of the present invention, and shall not be regarded as new content that has not been disclosed or anticipated herein, unless a person skilled in the art considers that the combination is obviously unreasonable.

## Claims

1. An acidic resin, wherein a copolymer in the acidic resin comprises a first structural unit derived from styrene having an electron-withdrawing group at the meta position of the benzene ring, a second structural unit derived from divinylbenzene, and a third structural unit derived from an allylsulfonic acid compound represented by Formula I;

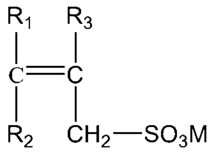
;

Formula 1

in Formula 1, $R_1$, $R_2$ and $R_3$ are each independently hydrogen or C1-C2 alkyl, and M is H or a metal element; wherein a portion of the benzene ring in the first structural unit carries a $-SO_3H$ group.

2. The acidic resin according to claim 1, **characterized in that**:
the copolymer in the acidic resin contains the first structural unit shown in Formula 2, the second structural unit shown in Formula 3, and the third structural unit shown in Formula 4,

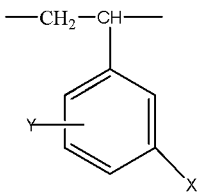
Formular 2 ;
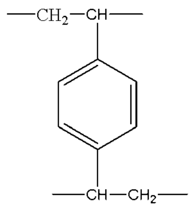
Formular 3 ;
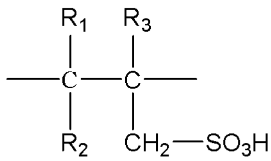
Formular 4 ;

wherein Y in Formula 2 is $-SO_3H$; and X is the electron-withdrawing group; and $R_1$, $R_2$ and $R_3$ in Formula 4 are each independently hydrogen or C1-C2 alkyl.

3. The acidic resin according to claim 1 or 2, **characterized in that**:

   the electron-withdrawing group is selected from the group consisting of halogen atom, nitro group, nitroso group, C1-C4 alkoxy, and C1-C4 alkanoyloxy group, for example acetoxy group; and/or,
   based on the total weight of the copolymer as 100wt%, the content of the first structural unit is 1-50wt%; the content of the second structural unit is 30-90wt%; and the content of the third structural unit is 1-50wt%; preferably, based on the total weight of the copolymer as 100wt%, the content of the first structural unit is 15-40wt%; the content of the second structural unit is 30-70wt%; and the content of the third structural unit is 10-45wt%.

4. The acidic resin according to any one of claims 1 to 3, **characterized in that**:

   the specific surface area of the acidic resin is 200-600 $m^2 \cdot g^{-1}$, preferably 200-500 $m^2 \cdot g^{-1}$; and/or,
   the pore volume of the acidic resin is 0.2-0.8 $cm^3 \cdot g^{-1}$, preferably 0.2-0.7 $cm^3 \cdot g^{-1}$; and/or,
   the pore size of the acidic resin is 1-20 nm, preferably 5-20 nm; and/or,
   the acid content of the acidic resin is 1-10 $mmol \cdot g^{-1}$, preferably 3-8 $mmol \cdot g^{-1}$.

5. The acidic resin according to any one of claims 1 to 4, **characterized in that** the acidic resin is used as a catalyst, preferably as a catalyst for the preparation of isosorbide from hexitol.

6. A method for preparing the acidic resin according to any one of claims 1 to 5, comprising the following steps:

   in the presence of a dispersant and an initiator, divinylbenzene, styrene having an electron-withdrawing group at the meta-position of the benzene ring, and an allylsulfonic acid compound represented by Formula I are

copolymerized to obtain a resin matrix;

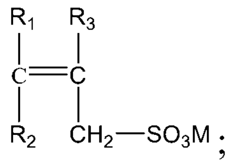

Formula 1

wherein $R_1$, $R_2$ and $R_3$ are each independently hydrogen or C1-C2 alkyl, and M is H or a metal element; and the resin matrix is sulfonated to obtain the acidic resin.

**7.** The preparation method according to claim 6, **characterized in that** the preparation method comprises the following steps:

(1) a solution containing the dispersant is mixed with a mixed solution containing divinylbenzene, styrene having an electron-withdrawing group at the meta position of the benzene ring, the allylsulfonic acid compound of Formula I, and the initiator, and subjected to polymerization reaction to provide the resin matrix;
(2) the resin matrix is mixed with an organic solvent containing a pore-enlarging agent to obtain a mixed solution containing the resin matrix; and,
(3) the mixed solution containing the resin matrix is contacted with a sulfonating agent for sulfonation, and optionally washed and/or dried to obtain the acidic resin.

**8.** The preparation method according to claim 6 or 7, **characterized in that**:

the dispersant is selected from at least one of polyvinyl alcohol and hydroxyethyl cellulose; preferably, polyvinyl alcohol having a number average molecular weight of 50000 to 100000; and/or,
the initiator is an azo compound and/or a peroxide, preferably at least one of benzoyl peroxide, phenylacetyl peroxide, potassium peroxide, and azobisisobutyronitrile; and/or,
the pore-enlarging agent is at least one of dichloromethane, dichloroethane and n-heptane.

**9.** The preparation method according to any one of claims 6 to 8, **characterized in that**:

relative to 100 parts by mass of all monomers, the amount of the initiator used is 0.2-6 parts by mass, preferably 0.5-4 parts by mass; and the amount of the dispersant used is 1-10 parts by mass; and/or,
relative to 100 parts by mass of the resin matrix, the amount of the pore enlarging agent used is 2-40 parts by mass, preferably 2-30 parts by mass.

**10.** The preparation method according to claim 7, **characterized in that**:

the conditions for the polymerization reaction in step (1) are: a temperature of 60-150 °C, and/or a time of 3-14h; and/or,
mixing the resin matrix with the organic solvent containing the pore-enlarging agent in step (2) includes raising the temperature of the mixed system to 30-100 °C at a rate of 0.1-3 °C/min, preferably to 40-100 °C; and/or,
the conditions for sulfonation in step (3) include: a temperature of 30-100 °C, preferably 40-100 °C, and/or a time of 5-20 h; and/or,
the sulfonating agent includes a strong acid, and the strong acid is selected from at least one of concentrated sulfuric acid, chlorosulfonic acid, oleum, and methanesulfonic acid.

**11.** Use of the acidic resin according to any one of claims 1 to 5 or the acidic resin prepared by the preparation method according to any one of claims 6 to 10 as a catalyst in the preparation of isosorbide.

**12.** A method for preparing isosorbide, comprising the following steps:

1) contacting hexitol with a solid acid catalyst to perform a primary dehydration reaction to obtain a primary dehydration reaction product mixture; and
2) removing the solid acid catalyst from the primary dehydration reaction product mixture, and contacting the

resulting mixture with an acidic resin to perform a secondary dehydration reaction to obtain a reaction product containing isosorbide;

wherein the acidic resin is the acidic resin according to any one of claims 1 to 5 or the acidic resin prepared by the preparation method according to any one of claims 6 to 10.

**13.** The method for preparing isosorbide according to claim 12, wherein:

hexitol is selected from sorbitol and/or mannitol; and/or,
hexitol is solid hexitol and/or a solution of hexitol; and/or,
the solid acid catalyst is selected from at least one of a molecular sieve, an acidic resin different from the acidic resin according to any one of claims 1 to 5 or the acidic resin prepared by the preparation method according to any one of claims 6 to 10, a metal phosphate, a Lewis acid, and a heteropolyacid, preferably at least one of a heteropolyacid and an acidic resin different from the acidic resin according to any one of claims 1 to 5 or the acidic resin prepared by the preparation method according to any one of claims 6 to 10; more preferably a heteropolyacid, and further more preferably a phosphotungstic acid solid acid catalyst.

**14.** The method for preparing isosorbide according to any one of claims 12-13, **characterized in that**:

in the primary dehydration reaction, the mass ratio of the solid acid catalyst and hexitol is (0.1-1.0): 100, preferably (0.5-6): 100, more preferably (1-5): 100; and/or,
the selectivity for 1,4-sorbitan in the primary dehydration reaction is greater than 80%, preferably equal to or greater than 85%, and more preferably equal to or greater than 90%; and/or,
in the secondary dehydration reaction, the mass ratio of the acidic resin catalyst and hexitol is (1-10): 100, preferably (2-6): 100; and/or,
the yield of isosorbide prepared by the secondary dehydration reaction is greater than 80%.

**15.** The method for preparing isosorbide according to any one of claims 12 to 14, **characterized in that**:

the conditions in step 1) include: a reaction temperature of 80-130 °C, and/or a time, such as a residence time, of 3-7 h, and/or a reaction pressure of 0.001-1 atm; and/or,
the conditions in step 2) include: a reaction temperature of 120-160 °C, and/or a time, such as a residence time of 1-9 h, and/or a reaction pressure of 0.001-1 atm.

**16.** The method for preparing isosorbide according to any one of claims 12 to 15, **characterized in that**:

the water generated in the primary dehydration reaction is discharged from the top of the reactor during the reaction, and the solid acid catalyst is removed from the primary dehydration reaction product mixture after it is discharged from the reactor, and then it enters the reactor of the secondary dehydration reaction for reaction; and/or,
the primary dehydration reaction product mixture is dehydrated before the secondary dehydration reaction; and/or,
the preparation method further comprises purifying the obtained reaction product containing isosorbide after the secondary dehydration reaction to obtain purified isosorbide, preferably the purification method is distillation and/or crystallization.

**17.** The method for preparing isosorbide according to any one of claims 12 to 16, wherein the preparation method is a continuous preparation method.

# INTERNATIONAL SEARCH REPORT

International application No.
**PCT/CN2023/132168**

## A. CLASSIFICATION OF SUBJECT MATTER

C08F257/00(2006.01)i; C08F257/02(2006.01)i; C08F212/36(2006.01)i; C08F2/18(2006.01)i; C08F2/20(2006.01)i; C08F8/36(2006.01)i; C07D493/04(2006.01)i; B01J31/08(2006.01)i; B01J31/10(2006.01)i

According to International Patent Classification (IPC) or to both national classification and IPC

## B. FIELDS SEARCHED

Minimum documentation searched (classification system followed by classification symbols)

C08F257/-; C08F212/-; C08F2/-; C08F8/-; C07D493/-; B01J31/-

Documentation searched other than minimum documentation to the extent that such documents are included in the fields searched

Electronic data base consulted during the international search (name of data base and, where practicable, search terms used)

CNABS, CNTXT, ENTXTC, VCN, ENTXT, DWPI, VEN, WOTXT, CNKI, 万方, WANFANG, ISI-WEB OF SCIENCE, STN: 中国石油化工, 中石化, 贺黎明, 杜超, 伊卓, 王瑞璞, 冯再兴, 刘青, 孙竹芳, 谢伦嘉, 赵思源, 酸性树脂, 离子交换树脂, 催化剂, 烯丙基磺酸, 丙烯磺酸, 二乙烯基苯, 氯苯乙烯, 溴苯乙烯, 乙酰氧基苯乙烯, 硝基苯乙烯, 甲氧基苯乙烯, 乙氧基苯乙烯, 烷氧基苯乙烯, 吸电子, 异山梨醇, 催化稳定性, 高温稳定性, 收率, acid resin, ion exchange resin, Catalyst, allyl sulfonate, allyl sulfonic acid, Propenylsulfonic acid, Propenylsulfonate, divinylbenzene, DVB, Styrene, Electron withdrawing, Isosorbide, Catalytic Stability, High Temperature Stability, Heat stability, yield

## C. DOCUMENTS CONSIDERED TO BE RELEVANT

| Category* | Citation of document, with indication, where appropriate, of the relevant passages | Relevant to claim No. |
|---|---|---|
| A | CN 112574229 A (CHINA PETROLEUM & CHEMICAL CORPORATION et al.) 30 March 2021 (2021-03-30)<br>description, embodiment 4 | 1-17 |
| A | CN 112642484 A (DANDONG MINGZHU SPECIAL TYPE RESIN CO., LTD.) 13 April 2021 (2021-04-13)<br>description, paragraphs 6-12 | 1-17 |
| A | CN 109261202 A (SHANXI INSTITUTE OF COAL CHEMISTRY, CHINESE ACADEMY OF SCIENCES) 25 January 2019 (2019-01-25)<br>description, paragraphs 15-36 | 1-17 |
| A | CN 114437099 A (CHINA PETROLEUM & CHEMICAL CORPORATION et al.) 06 May 2022 (2022-05-06)<br>description, paragraphs 18-63 | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 December 2023** | **19 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2023/132168

C. DOCUMENTS CONSIDERED TO BE RELEVANT

| Category* | Citation of document, with indication, where appropriate, of the relevant passages | Relevant to claim No. |
|---|---|---|
| A | CN 111346673 A (UNIVERSITY OF JINAN) 30 June 2020 (2020-06-30)<br>description, paragraphs 6-20 | 1-17 |
| A | GB 788883 A (EXXON RESEARCH AND ENGINEERING CO.) 08 January 1958 (1958-01-08)<br>claims 1-19 | 1-17 |
| A | GB 1538419 A (TOHO BESLON CO., LTD.) 17 January 1979 (1979-01-17)<br>description, page 1, line 1 to page 6, line 34 | 1-17 |
| A | LIU, Shenghuan et al. "Synthesis and Application of an Efficient and Stable Solid Acid Catalyst St-c-AS"<br>*Advanced Materials Research,* Vol. 306-307, 16 August 2011 (2011-08-16), pages 1508-1511 ISSN: 1662-8985,<br>page 1509, Experimental | 1-17 |

Form PCT/ISA/210 (second sheet) (July 2022)

**INTERNATIONAL SEARCH REPORT**
**Information on patent family members**

International application No.
**PCT/CN2023/132168**

| Patent document cited in search report | | | Publication date (day/month/year) | Patent family member(s) | | | Publication date (day/month/year) |
|---|---|---|---|---|---|---|---|
| CN | 112574229 | A | 30 March 2021 | CN | 112574229 | B | 29 March 2022 |
| CN | 112642484 | A | 13 April 2021 | CN | 112642484 | B | 04 July 2023 |
| CN | 109261202 | A | 25 January 2019 | | None | | |
| CN | 114437099 | A | 06 May 2022 | | None | | |
| CN | 111346673 | A | 30 June 2020 | | None | | |
| GB | 788883 | A | 08 January 1958 | | None | | |
| GB | 1538419 | A | 17 January 1979 | DE | 2725698 | A1 | 08 December 1977 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6864378 B2
- CN 104788465 A
- CN 204752581 U
- US 6639067 B1
- CN 103980286 A